(19) Europäisches Patentamt
European Patent Office
Office européen des brevets

(11) Veröffentlichungsnummer: **0 003 049**
**B1**

(12) **EUROPÄISCHE PATENTSCHRIFT**

(21) Anmeldenummer: **78101848.6**

(22) Anmeldetag: **23.12.78**

(51) Int. Cl.³: **C 07 D 249/08,**
A 01 N 43/64

(54) **Carbamoyl-triazolyl-O,N-acetale, Verfahren zu ihrer Herstellung und ihre Verwendung als Fungizide**

(30) Priorität: **07.01.78 DE 2800544**

(43) Veröffentlichungstag der Anmeldung:
**25.07.79 Patentblatt 79/15**

(45) Bekanntmachung des Hinweises auf die Patenterteilung:
**20.08.80 Patentblatt 80/17**

(84) Benannte Vertragsstaaten:
**BE CH DE FR GB IT NL SE**

(56) Entgegenhaltungen:
**DE - A - 2 600 799**

(73) Patentinhaber: **Bayer AG**
**Zentralbereich Patente, Marken und Lizenzen**
**D - 5090 Leverkusen 1, Bayerwerk (DE)**

(72) Erfinder: **Büchel, Karl Heinz, Prof. Dr.**
**Bergerheide 62**
**D - 5600 Wuppertal 1 (DE)**
**Krämer, Wolfgang, Dr.**
**Am Eckbusch 39/162**
**D - 5600 Wuppertal 1 (DE)**
**Brandes, Wilhelm, Dr.**
**Eichendorffstrasse 3**
**D - 5653 Leichlingen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 049 B1

# Carbamoyl-triazolyl-O,N-acetale, Verfahren zu ihrer Herstellung und ihre Verwendung als Fungizide

Die vorliegende Erfindung betrifft neue Carbamoyl-triazolyl-O,N-acetale, mehrere Verfahren zu ihrer Herstellung und ihre Verwendung als Fungizide.

Es ist bereits bekannt geworden, daß acylierte Triazolyl-O,N-acetale, wie insbesondere im Phenylteil substituierte 2-Alkylcarbonyloxy-3,3-dimethyl-1-phenoxy-1-(1,2,4-triazol-1-yl)-butane, gute fungizide Eigenschaften aufweisen (vergleiche DT—OS 2 600 799 [Le A 16 838]). Deren Wirkung ist jedoch, insbesondere bei niedrigen Aufwandmengen und -konzentrationen, nicht immer ganz befriedigend. Weiterhin ist allgemein seit längerer Zeit bekannt, daß Zink-äthylen-1,2-bisdithiocarbamidat ein gutes Mittel zur Bekämpfung von pilzlichen Pflanzenkrankheiten ist (vergleich Phytopathology *33*, 1113 (1963)). Jedoch ist dessen Einsatz nur beschränkt möglich, da es bei niedrigen Aufwandmengen und -konzentrationen teilweise wenig wirksam ist.

Es wurden nun als neue Verbindungen die Carbamoyl-triazolyl-O,N,-acetale der Formel

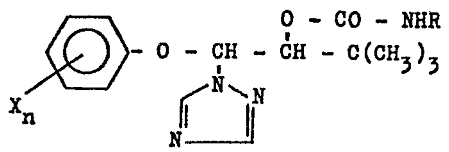

(I)

in welcher

R für Alkyl, Halogenalkyl, Alkoxycarbonyl, Alkoxyalkyl, substituiertes Phenyl oder Alkylsulfonylalkenyl-carbamoyl steht,

X für Halogen, Alkyl, Cycloalkyl, Alkoxy, Halogenalkyl, Alkylthio, Alkoxycarbonyl, gegebenenfalls substituiertes Phenyl oder Phenoxy, gegebenenfalls substituiertes Phenylalkyl, Amino, Cyano oder Nitro steht, und

n für ganze Zahlen von 0 bis 5 steht,

sowie deren physiologisch verträglichen Säureadditions-Salze und Metallsalz-Komplexe gefunden. Sie weisen starke fungizide Eigenschaften auf.

Die Verbindungen der Formel (I) besitzen zwei asymetrische Kohlenstoffatome; sie können deshalb in der erythro- wie in der threo-Form vorliegen. In beiden Fällen liegen sie vorwiegend als Racemate vor.

Weiterhin wurde nun gefunden, daß man die Carbamoyl-triazolyl-O,N-acetale der Formel (I) erhält, wenn man Triazolyl-Derivate der Formel

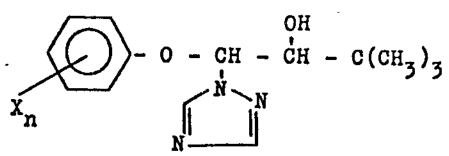

(II)

in welcher

X und n die oben angegebene Bedeutung haben,

a) mit Isocyanaten der Formel

$$O=C=N-R \qquad (III)$$

in welcher

R die oben angegebene Bedeutung hat,

in Gegenwart eines Lösungsmittels und gegebenenfalls in Gegenwart eines Katalysators umsetzt, oder

b) mit substituierten 1,3,4-Dioxazol-2-onen der Formel

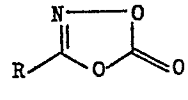

(IV)

in welcher

R die oben angegebene Bedeutung hat,

in Gegenwart eines Lösungsmittels und gegebenenfalls in Gegenwart eines katalysators umsetzt, oder

Weiterhin können die erfindungsgemäß erhältlichen Carbamoyl-triazolyl-O,N-acetale der Formel (I) durch Umsetzen mit Säuren in die Salze überführt werden, bzw. können durch Reaktion mit Metallsalzen die entsprechenden Metallkomplexe erhalten werden.

Ueberraschenderweise zeigen die erfindungsgemäßen Carbamoyl-triazolyl-O,N-acetale eine erheblich höhere fungizide Wirksamkeit als die aus dem Stand der Technik bekannten acylierten Triazolyl-O,N-acetale, wie insbesondere im Phenylteil substituierte 2-Alkylcarbonyloxy-3,3-dimethyl-1-phenoxy-1-(1,2,4-triazol-1-yl)-butane, welche chemisch und wirkungsmäßig naheliegendste Verbindungen sind, und als das Zink-äthylen-1,2-bisdithiocarbamidat, welches ein bekannter Stoff gleicher Wirkungsrichtung ist.

Verwendet man 1-(4-Biphenylyloxy)-3,3-dimethyl-1-(1,2,4-triazol-1-yl)-butan-2-ol und Methoxymethylisocyanat als Ausgangsstoffe, so kann der Reaktionsablauf durch das folgende Formelschema wiedergegeben werden (Verfahren a):

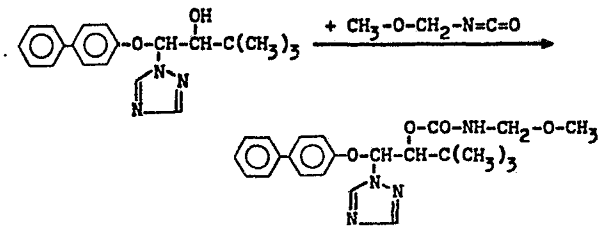


Verwendet man 1-(4-Biphenylyloxy)-3,3-dimethyl-1-(1,2,4-triazol-1-yl)-butan-2-ol und 5-Methoxymethyl-1,3,4-dioxazol-2-on als Ausgangsstoffe, so kann der Reaktionsablauf durch das folgende Formelschema wiedergegeben werden (Verfahren b):

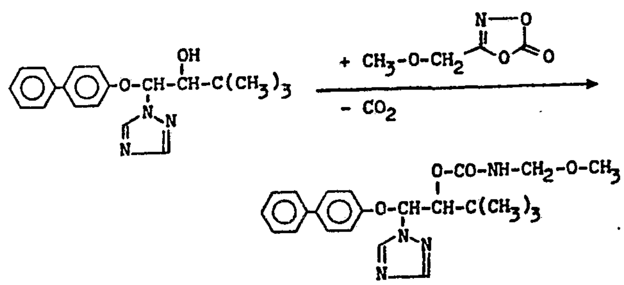


Die als Ausgangsstoffe zu verwendenden Triazolyl-Derivate sind durch die Formel (II) allgemein definiert. In dieser Formel steht X vorzugsweise für Halogen, Amino, Cyano, Nitro, geradkettiges oder verzweigtes Alkyl mit bis zu 4 Kohlenstoffatomen, Cycloalkyl mit 5 bis 7 Kohlenstoffatomen, wie insbesondere Cyclohexyl, Halogenalkyl mit bis zu 2 Kohlenstoff- und bis zu 5 Halogenatomen, wie insbesondere Fluor- und Chloratomen; weiterhin vorzugsweise für Alkoxycarbonyl mit insgesamt bis zu 5 Kohlenstoffatomen, Alkoxy und Alkylthio mit jeweils bis zu 2 Kohenstoffatomen. X steht außerdem vorzugsweise für gegebenenfalls substituiertes Phenyl und Phenoxy, wobei als Substituenten vorzugsweise in Frage kommen: Halogen, Amino, Cyano, Nitro oder Alkyl mit 1 bis 2 Kohlenstoffatomen; schließlich noch vorzugsweise für gegebenenfalls substituiertes Phenylalkyl mit 1 oder 2 Kohlenstoffatomen im Alkylteil, wobei als Substituenten im Alkylteil vorzugsweise Alkylcarbonyloxy mit insgesamt bis zu 3 Kohlenstoffatomen und als Substituenten im Phenylteil vorzugsweise Halogen, Nitro und Cyano genannt sein sollen.

Der Index n steht vorzugsweise für die Zahlen 0 bis 3.

Die Ausgangsstoffe der Formel (II) sind bekannt (vgl. Deutsche Offenlegungsschrift 2 324 010 [LeA 14 971]). Noch nicht bekannte Ausgangsstoffe der Formel (II) können nach den schon beschriebenen Verfahren erhalten werden, indem man z.B. die entsprechenden Keton-Derivate mit Aluminiumisopropylat oder mit komplexen Hydriden in Gegenwart eines Lösungsmittels reduziert.

Die für die Verfahrenswariante (a) als Ausgangsstoffe zu verwendenden Isocyanate sind durch die Formel (III) allgemein definiert. In dieser Formel steht R vorzugsweise für geradkettiges oder verzweigtes Alkyl mit 5 bis 12 Kohlenstoffatomen, Halogenalkyl mit bis zu 4 Kohlenstoff- und bis zu 5 gleichen oder verschiedenen Halogenatomen, wie insbesondere Fluor- und Chloratomen, sowie für Alkoxycarbonyl und Alkoxyalkyl mit jeweils 1 bis 4 Kohlenstoffatomen in jedem Alkylteil. R steht außerdem vorzugsweise für einfach oder mehrfach substituiertes Phenyl, wobei als Substituenten vorzugsweise in Frage kommen: geradkettiges oder verzweigtes Alkyl mit 1 bis 4 Kohlenstoffatomen, Alkoxy und Alkylthio mit jeweils 1 oder 2 Kohlenstoffatomen, Halogenalkyl mit bis zu 2 Kohlenstoff- und bis zu 5 gleichen oder verschiedenen Halogenatomen, wie insbesondere Fluor- und Chloratomen sowie Alkoxycarbonylalkenyl mit 1 bis 4 Kohlenstoffatomen im Alkylteil und 2 bis 4 Kohlenstoffatomen im Alkenylteil. R steht ferner vorzugsweise für Alkylsulfonyl-alkenyl-carmaboyl mit 1 bis 4 Kohlenstoffatomen im Alkylteil und 2 bis 4 Kohlenstoffatomen im Alkenylteil.

Die Isocyanate der Formel (III) sind bekannt oder lassen sich nach allgemein üblichen und bekannten Verfahren herstellen; so z.B. durch Umsetzung von Aminen mit Phosgen und anschliessendem Erhitzen.

Die für die Verfahrensvariante (b) als Ausgangsstoffe zu verwendenden 1,3,4-Dioxazol-2-one sind durch die Formel (IV) allgemein definiert. In dieser Formel steht R vorzugsweise für die Reste, die bei den Isocyanaten der Formel (III) bereits vorzugsweise genannt wurden.

Die 1,3,4-Dioxazol-2-one der Formel (IV) sind bekannt (vgl. G. Beck, Chem. Ber, *84*, 688 (1951)) oder lassen sich nach allgemein üblichen und bekannten Verfahren herstellen; so z.B. durch Umsetzung entsprechender Hydroxycarbonsäuren oder Säurehydrazide mit Phosgen in der Siedehitze.

Als Lösungsmittel kommen für die Umsetzung gemäß Verfahrensvariante (a) vorzugsweise alle inerten organischen Lösungsmittel in Frage. Hierzu gehören vorzugsweise Ketone, wie Diäthylketon, insbesondere Aceton und Methyläthylketon; Nitrile, wie Propionitril, insbesondere Acetonitril; Aether, wie Tetrahydrofuran oder Dioxan; Ester, wie Essigsäureäthylester; aromatische Kohlenwasserstoffe, wie Benzol oder Toluol und halogenierte Kohlenwasserstoffe, wie Methylenchlorid, Tetrachlorkohlenstoff oder Chloroform.

Als Katalysatoren können beim Verfahren (a) vorzugsweise verwendet werden: tertiäre Basen, wie Triäthylamin und Pyridin oder Zinn-organische Verbindungen, wie Dibutylzinndilaurat.

Die Reaktionstemperaturen können bei der Durchführung der Verfahrensvariante (a) in einem größeren Bereich variiert werden. Im allgemeinen arbeitet man zwischen 0 und 100°C, vorzugsweise Zwischen 20 und 70°C.

Bei der Durchführung der Verfahrensvariante (a) arbeitet man vorzugsweise in molaren Mengen. Zur Isolierung der Verbindungen der Formel (I) wird das Lösungsmittel abdestilliert und der Rückstand nach üblichen Methoden aufgearbeitet.

Als Lösungsmittel kommen für die Umsetzung gemäß Verfahrensvariante (b) vorzugsweise inerte organische Lösungsmittel in Frage. Hierzu gehören vorzugsweise die bei der Verfahrensvariante (a) bereits genannten Solventien.

Als Katalysatoren können beim Verfahren (b) vorzugsweise verwendet werden: tertiäre Amine, wie beispielsweise Triäthylamin, oder Alkalisalze von Fettsäuren, wie beispielsweise Natriumacetat.

Die Reaktionstemperaturen können bei der Durchführung der Verfahrensvariante (b) in einem größeren Bereich variiert werden. Im allgemeinen arbeitet man zwischen 60 und 150°C, vorzugsweise zwischen 80 und 100°C.

Bei der Durchführung der Verfahrensvariante (b) arbeitet man vorzugsweise in molaren Mengen. Zur Isolierung der Verbindungen der Formel (I) wird das Lösungsmittel abdestilliert und der Rückstand nach üblichen Methoden aufgearbeitet.

Zur Herstellung von Säureadditionssalzen der Verbindungen der Formel (I) kommen alle physiologische verträglichen Säuren in Frage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV-Haupt- und der I. und II. sowie IV. bis VIII. Nebengruppe in Frage, wobei Kupfer, Zink, Mangan, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z.B. durch Lösen des Metallsalzes in Alkohol, z.B. Aethanol und Hinzufügen zur Verbindung der Formel (I). Man kann Metallsalzkomplexe in bekannter Weise, z.B. durch Abfiltrieren, isolieren und gegebenenfalls durch Umkristallisation reinigen.

Als Beispiele für besonders wirksame Vertreter der erfindungsgemäßen Wirkstoffe seien außer den Herstellungsbeispielen und den Beispielen der Tabelle 1 gennant:

1 - (4 - Chlorphenoxy) - 3,3 - dimethyl - 2 - methoxymethyl - carbamoyloxy - 1 - (1,2,4 - triazol - 1 - yl) - butan

1 - (2,4 - Dichlorphenoxy) - 3,3 - dimethyl - 2 - methoxymethyl - carbamoyloxy - 1 - (1,2,4 - triazol - 1 - yl) - butan

1 - (4 - Chlorphenoxy) - 3,3 - dimethyl - 2 - trifluormethylcarbamoyloxy - 1 - (1,2,4 - triazol - 1 - yl) - butan

1 - (4 - Biphenylyloxy) - 3,3 - dimethyl - 2 - trifluormethylcarbamoyloxy - 1 - (1,2,4 - triazol - 1 - yl) - butan

Die erfindungsgemäßen Wirkstoffe weisen eine starke fungi — toxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen und die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung von Venturia-Arten, wie z.B. gegen den Erreger des Apfelschorfs (Fusicladium dendriticum), von Uromyces-Arten, wie z.B. den Erreger des Bohnenrostes (Uromyces phaseoli), sowie zur Bekämpfung von Phytophthora-Arten und Getreidekrankheiten verwendet werden.

Als Pflanzenschutzmittel können die erfindungsgemäßen Wirkstoffe zur Saatgut- oder Bodenbehandlung und zur Behandlung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Anwendung geschieht in üblicher Weise, z.B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentrationen in den Anwendungsformen in einem größeren Bereich variiert werden. Sie liegen im allgemeinen zwischen 0,1 und 0,00001 Gewichtsprozent. Vorzugsweise zwischen 0,05 und 0,0001%.

Bei der Saatgutbehandlung werden im allgemeinen Wirkstoffmengen von 0,001 bis 50 g je Kilogramm Saatgut, vorzugsweise 0,01 bis 10 g benötigt.

Zur Bodenbehandlung sind Wirkstoffmengen von 1 bis 1000 g je cbm Boden, wie insbesondere 10 bis 200 g, erforderlich.

Die vielseitigen Verwendungsmöglichkeiten gehen aus den nachfolgenden Beispielen hervor.

Beispiel A

Fusicladium-Test (Apfel) / Protektiv

Lösungsmittel: 4,7 Gewichtsteile Aceton
Emulgator: 0,3 Gewichtsteile Alkyl-aryl-polygykoläther
Wasser: 95,0 Gewichtsteile

Man vermischt die für die gewünschte Wirkstoffkonzentration in der Spritzflüssigkeit nötigen Wirkstoffmenge mit der angegebenen Menge des Lösungsmittels und verdünnt das Konzentrat mit der angegebenen Menge Wasser, welches die gennanten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie mit einer wässrigen Konidiensuspension des Apfelschorferregers (Fusicladium dentriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

In diesem Test zeigen z.B., folgende Verbindungen eine sehr gute Wirkung, die derjenigen der aus dem Stand der Technik bekannten Verbindungen deutlich überlegen ist: Verbindungen gemäß Herstellungsbeispielen 1, 2, 8, 16, 17.

Beispiel B

Uromyces-Test (Bohnen) / Protektiv

Lösungsmittel: 4,7 Gewichtsteile Aceton
Emulgator: 0,3 Gewichtsteile Alkyl-aryl-polyglykoläther
Wasset: 95,0 Gewichtsteile

Man vermischt die für gewünschte Wirkstoffkonzentration in der Spritzflüssigkeit notwendige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Bohnenpflanzen, die sich im 2-Blattstadium befinden, bis zur Tropfnässe. Die Pflanzen verbleiben zum Abtrocknen 24 Stunden bei 20—22°C und einer relativen Luftfeuchtigkeit von 70% im Gewächshaus. Anschließend werden sie mit einer wässrigen Uredosporensuspension des Bohnenrosterregers (Uromyces phaseoli) inokuliert und 24 Stunden lang in einer dunklen Feuchtkammer bei 20—22°C und 100% relativer Luftfeuchtigkeit inkubiert.

Die Pflanzen werden dann unter intensiver Belichtung für 9 Tage bei 20—22°C und einer relativen Luftfeuchtigkeit von 70—80% im Gewächshaus aufgestellt.

10 Tage nach der Inokulation wird der Befall der Pflanzen bestimmt. Die erhaltenen Boniturwerte werden in Prozent Befall umgerechnet. 0% bedeutet keinen Befall, 100% bedeutet, daß die Pflanzen vollständig befallen sind.

In diesem Test zeigen z.B. folgende Verbindungen eine sehr gute Wirkung, die derjenigen der aus dem Stand der Technik bekannten Verbindungen deutlich überlegen ist.

Verbindungen gemäß Herstellungsbeispielen 1, 2, 3, 4, 5, 6, 7, 8, 9, 10, 11, 13, 14, 15, 16, 17.

Beispiel C

Phytophthora-Test (Tomaten) / Protektiv

Lösungsmittel: 4,7 Gewichtsteile Aceton
Emulgator 0,3 Gewichtsteile Alkyl-aryl-polyglykoläther
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20°C und einer relativen Luftfeuchtigkeit von 70% im Gewächshaus. Anschließend werden die Tomatenpflanzen mit einer wässrigen Sporensuspension von Phytophthora infestans inokuliert. Die Pflanzen werden in eine Feuchtkammer mit einer 100%igen Luftfeuchtigkeit und einer Temperatur von 18 bis 20°C gebracht.

Nach 5 Tagen wird der Befall der Tomatenpflanzen bestimmt. Die erhaltenen Boniturwerte werden auf Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt.

In diesem Test zeigen z.B. folgende Verbindungen eine sehr gute Wirkung, die derjenigen der aus dem Stand der Technik bekannten Verbindungen deutlich überlegen ist: Verbindungen gemäß Herstellungsbeispielen 1, 5, 8, 16, 17.

Herstellungsbeispiele
Beispiel 1

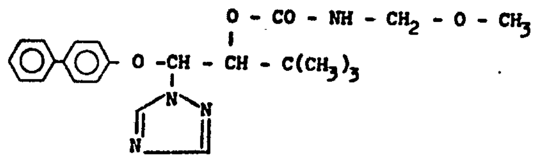

(Verfahren a)

505g (1,5 Mol) 1-(4-Biphenylyloxy)-3,3-dimethyl-1-(1,2,4-triazol-1-yl)-butan-2-ol (A-Form) und 140g (1,6 Mol) Methoxymethylisocyanat werden in 2,5 l Tetrahydrofuran in Gegenwart von 40 ml Triäthylamin und 1 ml Dibutyl-zinn-dilaurat 48 Stunden unter Rückfluß erhitzt. Danach wird das Lösungsmittel im Vakuum abgezogen und der Rückstand mit 3 l Petroläther versetzt. Der entstehende kristalline Niederschlag wird mit 1,5 l Diisopropyläther ausgerührt. Man erhält 528g (82,5% der Theorie) 1-(4-Bisphenylyloxy)-3,3-dimethyl-2-methoxymethylcarbamoyloxy-1-(1,2,4-triazol-1-yl)-butan (Form A) vom Schmelzpunkt 95—98°C.

Analog werden die Beispiele der folgenden Tabelle 1 erhalten.

Tabelle 1

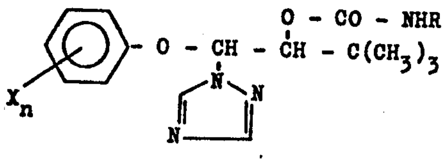

| Bsp. Nr. | $X_n$ | R | Schmelzpunkt (°C) |
|---|---|---|---|
| 2 | 4-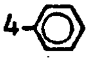 | $-(CH_2)_2-Cl$ | 138–41 (B-Form) |
| 3 | 4-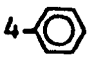 | $-CO-OCH_3$ | 184–85 (B-Form) |
| 4 | 4-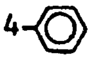 | $-CO-OC_2H_5$ | 164–66 (B-Form) |
| 5 | 4-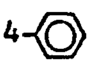 | $-(CH_2)_2-Cl$ | 103–05 (A-Form) |
| 6 | 4-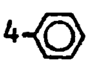 | $-CO-OCH_3$ | 120 (A-Form) |
| 7 | 4-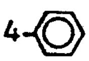 | $-CO-OC_2H_5$ | 100 (A-Form) |
| 8 | 4-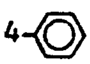 | $-CH_2-O-CH_3$ | 141–44 (B-Form) |
| 9 | 4-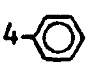 | $-CO-N(SO_2CH_3)-CH_2-CH=CH_2$ | 141–44 (B-Form) |
| 10 | 4-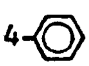 | $-(CH_2)_2-CH(CH_3)-CH_2-C(CH_3)_3$ | 109–15 (B-Form) |
| 11 | 4-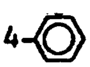 | 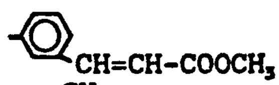 | 194–200 |
| 12 | 4-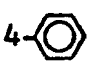 | 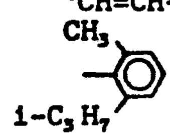 | 158–65 |
| 13 | 4-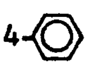 | 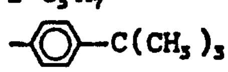 | 193–206 |
| 14 | 4-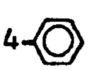 | 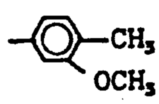 | 148–55 |
| 15 | 4-Cl | $-CO-OCH_3$ | 151–54 (A-Form) |
| 16 | 4-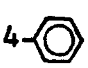 | $-CH_2-O-C_2H_5$ | 140(xHCl) |
| 17 | 4-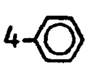 | $-(CH_2)_3-O-CH_3$ | 100(xHCl |

A- und B-Form = jeweils eine der beiden möglichen geometrischen Isomeren.

**Patentansprüche**

1. Carbamoyl-triazolyl-O,N-acetale der allgemeinen Formel

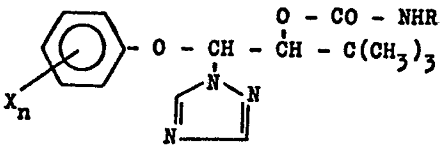 (I)

in welcher
R für Alkyl, Halogenalkyl, Alkoxycarbonyl, Alkoxyalkyl, substituiertes Phenyl oder Alkylsulfonyl-alkenyl-carbamoyl steht,
X für Halogen, Alkyl, Cycloalkyl, Alkoxy, Halogenalkyl, Alkylthio, Alkoxycarbonyl, gegebenenfalls substituiertes Phenyl oder Phenoxy, gegebenenfalls substituiertes Phenylalkyl, Amino, Cyano oder Nitro steht, und
n für ganze Zahlen von 0 bis 5 steht,
sowie deren Säureadditions-Salze und Metallsalz-Komplexe.

2. Verfahren zur Herstellung von Carbamoyl-triazolyl-O,N-acetalen, dadurch gekennzeichnet, daß man Triazolyl-Derivate der Formel

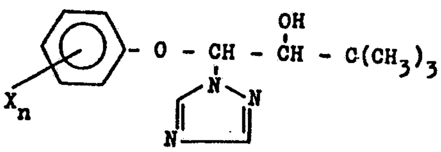 (II)

in welcher
X und n die in Anspruch 1 angegebene Bedeutung haben,
a) mit Isocyanaten der Formel

$$O{=}C{=}N{-}R \qquad \text{(III)}$$

in welcher
R die in Anspruch 1 angegebene Bedeutung hat,
in Gegenwart eines Lösungsmittels und gegebenenfalls in Gegenwart eines Katalysators umsetzt, oder
b) mit substituierten 1,3,4-Dioxazol-2-onen der Formel

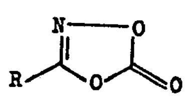 (IV)

in welcher
R die in Anspruch 1 angegebene Bedeutung hat,
in Gegenwart eines Lösungsmittels und gegebenenfalls in Gegenwart eines Katalysators umsetzt, und gegebenenfalls durch Addition von Säuren oder Metallsalzen die entsprechenden Salze bzw. Metallkomplexe herstellt.

3. Fungizide Mittel, gekennzeichnet durch einen Gehalt an mindestens einem Carbamoyl-triazolyl-O,N-acetal gemäß Anspruch 1.

4. Verwendung von Carbamoyl-triazolyl-O,N-acetalen gemäß Anspruch 1 zur Bekämpfung von Pilzen.

**Revendications**

1. Carbamoyl-triazolyl-O,N-acétals de formule générale:

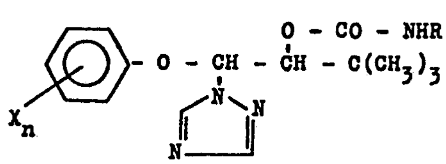 (I)

dans laquelle
R représente un alcoyle, haloalcoyle, alcoxycarbonyle, alcoxyalcoyle, phényle substitué ou alcoylsulfonylalcényl-carbamoyle,
X représente de l'halogène, un alcoyle, cycloalcoyle, alcoxy, haloalcoyle, alcoylthio, alcoxy-

carbonyle, phényle ou phénoxy éventuellement substitué, phénylalcoyle éventuellement substitué, amino, cyano ou nitro et

n représente des nombres entiers de 0 à 5,

ainsi que leurs sels d'addition d'acides et complexes de sels métalliques.

2. Procédé de préparation de carbamoyl-triazolyl-O,N-acétals, caractérisé en ce qu'on fait réagir des dérivés de triazolyle de formule

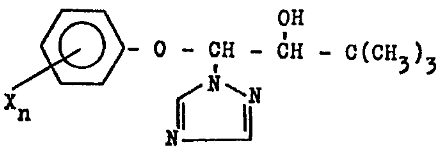

(II)

dans laquelle

X et n ont la signification indiquée à la revendication 1,

a) avec des isocyanates de formule:

O=C=N—R (III)

dans laquelle

R a la signification indiquée à la revendication 1, en présence d'un solvant et éventuellement en présence d'un catalyseur, ou

b) avec des 1,3,4-dioxazol-2-ones substituées de formule:

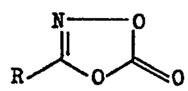

(IV)

dans laquelle

R a la signification indiquée à la revendication 1,

en présence d'un solvant et éventuellement en présence d'un catalyseur, et en ce qu'éventuellement par addition d'acides ou de sels métalliques on prépare les sels ou complexes métalliques correspondants.

3. Agents fongicides, caractérisés par une teneur en au moins un carbamoyl-triazolyl-O,N-acétal selon la revendication 1.

4. Utilisation de carbamoyl-triazolyl-O,N-acétals selon la revendication 1 pour combattre les champignons.

**Claims**

1. Carbamoyl-triazolyl-O,N-acetals of the general formula

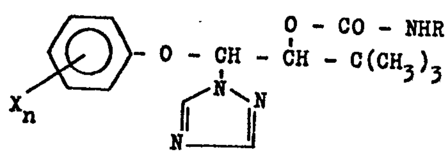

(I)

in which

R represents alkyl, halogenoalkyl, alkoxycarbonyl, alkoxyalkyl, substituted phenyl or alkylsulphonylalkenyl-carbamoyl,

X represents halogen, alkyl, cycloalkyl, alkoxy, halogenoalkyl, alkylthio, alkoxycarbonyl, optionally substituted phenyl or phenoxy, optionally substituted phenylalkyl, amino, cyano or nitro, and

n represents integers from 0 to 5,

and their acid-addition salts and metal-salt complexes.

2. A process for the preparation of carbamoyl-triazolyl-O,N-acetals, characterised in that triazolyl derivatives of the formula

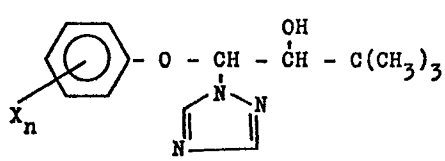

(II)

in which

X and n have the meanings stated in claim 1, are reacted
a) with isocyanates of the formula

$$O{=}C{=}N{-}R \quad (III)$$

in which
R has the meaning stated in claim 1,
in the presence of a solvent and optionally in the presence of a catalyst, or
b) with substituted 1,3,4-dioxazol-2-ones of the formula

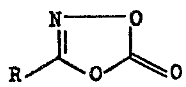

(IV)

in which
R has the meaning stated in claim 1,
in the presence of a solvent and optionally in the presence of a catalyst, and, optionally, by the addition of acids or metal salts, the corresponding salts or metal complexes are prepared.

3. Fungicidal compositions, characterised in that they contain at least one carbamoyl-triazolyl-O,N-acetal according to claim 1.

4. The use of carbamoyl-triazolyl-O,N-acetals according to claim 1 for combating fungi.